# EUROPEAN PATENT APPLICATION

(11) **EP 3 363 440 A1**
(43) Date of publication of application: **22.08.2018**
(21) Application number: 17386004.0
(22) Date of filing: 21.02.2017
(51) Int. Cl.: A61K 31/575, A61P 1/02, A61K 36/22

(54) **INHIBITION OF ORAL MICROBIAL GROWTH BY A COMPOUND OF PISTACIA LENTISCUS**

(71) Applicant: University Of Athens, 10679 Athens (GR); Pharmagnose S.A., 34100 Eyboia (GR); Albert-Ludwigs-Universität Freiburg, 79085 Freiburg (DE)
(72) Inventor: Skaltsounis, Leandros, 15127 Melissia Attikis (GR); Argyropoulou, Aikaterini, 15127 Melissia Attikis (GR); Karygianni, Lamprini, 79106 Freiburg (DE); AL-Ahmad, Ali, 79117 Freiburg (DE); Hellwig, Elmar, 79102 Freiburg (DE)
(74) Representative: Roukounas, Dimitrios

(57) **Abstract**

Use of 24Z-isomasticadienolic acid or a pharmaceutically acceptable salt thereof in the prevention or treatment of oral infections caused by microbial growth in the oral cavity.

## Description

### Field of the invention

The present invention relates to the prevention or treatment of oral infections caused by microbial growth in the oral cavity.

### Background of the invention

Therapies of various bacterially-driven infections based on herbal agents are one of the most current therapeutic trends in medicine (Mahady, G.B. (2005). Medicinal plants for the prevention and treatment of bacterial infections. Curr Pharm Des 11, 2405-2427). Nevertheless, more than 300.000 plant extracts need to be screened for their antioxidant and antimicrobial properties (Sofrata, A., Santangelo, E.M., Azeem, M., Borg-Karlson, A.K., Gustafsson, A., and Putsep, K. (2011). Benzyl isothiocyanate, a major component from the roots of Salvadora persica is highly active against Gram-negative bacteria. PLoS One 6, e23045; Ngo, L.T., Okogun, J.I., and Folk, W.R. (2013). 21st century natural product research and drug development and traditional medicines. Nat Prod Rep 30, 584-592; Takebe, Y., Saucedo, C.J., Lund, G., Uenishi, R., Hase, S., Tsuchiura, T., Kneteman, N., Ramessar, K., Tyrrell, D.L., Shirakura, M., Wakita, T., Mcmahon, J.B., and O'keefe, B.R. (2013). Antiviral lectins from red and blue-green algae show potent in vitro and in vivo activity against hepatitis C virus. PLoS One 8, e64449). Among these, a selection of Mediterranean plant extracts from olive (*Olea europaea),* parsley (*Petroselinum crispum*), oregano (*Origanum vulgare*), thyme (*Thymus vulgaris*), sage (*Salvia officinalis*), mastic gum (*Pistacia lentiscus*) and false yellowhead (*Inula viscosa*) have shown a significant inhibitory activity against numerous bacteria (Wahba, N.M., Ahmed, A.S., and Ebraheim, Z.Z. (2010). Antimicrobial effects of pepper, parsley, and dill and their roles in the microbiological quality enhancement of traditional Egyptian Kareish cheese. Foodborne Pathog Dis 7, 411-418; Petrolini, F.V., Lucarini, R., De Souza, M.G., Pires, R.H., Cunha, W.R., and Martins, C.H. (2013). Evaluation of the antibacterial potential of Petroselinum crispum and Rosmarinus officinalis against bacteria that cause urinary tract infections. Braz J Microbiol 44, 829-834; Fournomiti, M., Kimbaris, A., Mantzourani, I., Plessas, S., Theodoridou, I., Papaemmanouil, V., Kapsiotis, I., Panopoulou, M., Stavropoulou, E., Bezirtzoglou, E.E., and Alexopoulos, A. (2015). Antimicrobial activity of essential oils of cultivated oregano (Origanum vulgare), sage (Salvia officinalis), and thyme (Thymus vulgaris) against clinical isolates of Escherichia coli, Klebsiella oxytoca, and Klebsiella pneumoniae. Microb Ecol Health Dis 26, 23289). However, to elucidate the mechanisms relating to their complex biological behavior, the effect of pure plant-derived compounds on microorganisms has to be investigated (Alvin, A., Miller, K.I., and Neilan, B.A. (2014). Exploring the potential of endophytes from medicinal plants as sources of antimycobacterial compounds. Microbiol Res 169, 483-495; Tiwari, V., Roy, R., and Tiwari, M. (2015). Antimicrobial active herbal compounds against Acinetobacter baumannii and other pathogens. Front Microbiol 6, 618).

The antimicrobial traits of plants can be attributed to the activity of natural antibiotics with low molecular weight (MW < 500), named phytoalexins, and their synergistic action as well ( Gonzalez-Lamothe, R., Mitchell, G., Gattuso, M., Diarra, M.S., Malouin, F., and Bouarab, K. (2009). Plant antimicrobial agents and their effects on plant and human pathogens. Int J Mol Sci 10, 3400-3419; Ng, T.B., Ye, X.J., Wong, J.H., Fang, E.F., Chan, Y.S., Pan, W., Ye, X.Y., Sze, S.C., Zhang, K.Y., Liu, F., and Wang, H.X. (2011). Glyceollin, a soybean phytoalexin with medicinal properties. Appl Microbiol Biotechnol 90, 59-68). These well-studied antimicrobial stress-derived metabolites of plant origin include flavonoids, glycosteroids, terpenoids, and polyphenols (Ahuja, I., Kissen, R., and Bones, A.M. (2012). Phytoalexins in defense against pathogens. Trends Plant Sci 17, 73-90). Furthermore, other specific defense mechanisms of plants are supported by the release of avirulence (Avr) gene-activated resistance (R) proteins or the secretion of a polysaccharide with (1-3)-ß-D-glucan subunits, namely callose, under the threat of the microbial invaders (Dangl, J.L., and Jones, J.D. (2001). Plant pathogens and integrated defence responses to infection. Nature 411, 826-833; Maor, R., and Shirasu, K. (2005). The arms race continues: battle strategies between plants and fungal pathogens. Curr Opin Microbiol 8, 399-404). Finally, plants produce endogenous antimicrobial peptides with less than 100 amino acid residues, low acquisition of resistance and broad-spectrum antimicrobial features (Abedinzadeh, M., Gaeini, M., and Sardari, S. (2015). Natural antimicrobial peptides against Mycobacterium tuberculosis. J Antimicrob Chemother 70, 1285-1289).

An integral part of the healthy human body are trillions of microbes which outnumber host cells by 10 to 1 (Hoffmann, A.R., Proctor, L.M., Surette, M.G., and Suchodolski, J.S. (2015). The Microbiome: The Trillions of Microorganisms That Maintain Health and Cause Disease in Humans and Companion Animals. Vet Pathol). Although microbes could benefit humans by carrying 8 million health-related genes, they are also able to turn into pathogenic body inhabitants under specific circumstances (Ding, T., and Schloss, P.D. (2014). Dynamics and associations of microbial community types across the human body. Nature 509, 357-360; Blekhman, R., Goodrich, J.K., Huang, K., Sun, Q., Bukowski, R., Bell, J.T., Spector, T.D., Keinan, A., Ley, R.E., Gevers, D., and Clark, A.G. (2015). Host genetic variation impacts microbiome composition across human body sites. Genome Biol 16, 191). The high pathogenicity of bacteria, viruses, and fungi can be demonstrated through the formation of antibiotic-resistant oral biofilms (Battin, T.J., Sloan, W.T., Kjelleberg, S., Daims, H., Head, I.M., Curtis, T.P., and Eberl, L. (2007). Microbial landscapes: new paths to biofilm research. Nat Rev Microbiol 5, 76-81).

The healthy oral cavity comprises more than 700 different bacterial (Gram-positive, Gram-negative), viral, and fungal species (Avila, M., Ojcius, D.M., Yilmaz, O. (2009). The oral microbiota: living with a permanent guest. DNA Cell Biol 28, 405-411). The most prevalent microorganisms in healthy communities are *Streptococcus* spp. (*Streptococcus mutans, Streptococcus sobrinus, Streptococcus oralis*), *Fusobacterium* spp. (*Fusobacterium nucleatum*), *Porphyromonas* spp. (*Porphyromonas gingivalis*), *Parvimonas* spp. (*Parvimonas micra*), *Lactobacterium* spp., *Actinomyces* spp., *Prevotella* spp., *Treponema* spp., *Veillonella* spp., *Staphylococcus* spp. (*Staphylococcus aureus*), and *Propionibacterium* spp. (Jenkinson, H.F., Lamont, R.J. (2005). Oral microbial communities in sickness and in health. Trends Microbiol 13, 589-595). *Enterococcus faecalis* is mostly recovered from root-filled teeth with persistent periapical lesions (Karygianni, L., Wiedmann-Al-Ahmad, M., Finkenzeller, G., Sauerbier, S., Wolkewitz, M., Hellwig, E., Al-Ahmad, A. (2012). Enterococcus faecalis affects the proliferation and differentiation of ovine osteoblast-like cells. Clin Oral Investig 16, 879-887). Interestingly, these commensal oral microbes live in symbiosis with the host and keep the pathogenic species under control (Paster, B.J., Olsen, I., Aas, J.A., Dewhirst, F.E. (2006). The breadth of bacterial diversity in the human periodontal pocket and other oral sites. Periodontol 2000 42, 80-87). Under pathogenic conditions oral bacteria are able to form biofilms consisting of bacterial cells enmeshed in a polysaccharide-rich extracellular matrix (Keijser, B. J., E. Zaura, S. M. Huse, J. M. van der Vossen, F. H. Schuren, R. C. Montijn, J. M. ten Cate, and W. Crielaard. (2008). Pyrosequencing analysis of the oral microflora of healthy adults. J Dent Res 87, 1016-1020). As a result, biofilm-associated oral diseases such as caries, gingivitis or periodontitis can occur (Beikler, T., and Flemmig, T.F. (2011). Oral biofilm-associated diseases: trends and implications for quality of life, systemic health and expenditures. Periodontol 2000 55, 87-103).

24Z-Isomasticadienolic acid is a known compound, isolated from *Pistacia lentiscus* L. (Anacardiaceae). It has been reported that it exhibits antibacterial properties against *Helicobacter pylori* strains (Paraschos, S., Magiatis, P., Mitakou, S., Petraki, K., Kalliaropoulos, A., Maragkoudakis, P., Mentis, A., Sgouras, D., and Skaltsounis, A.L. (2007). In vitro and in vivo activities of Chios mastic gum extracts and constituents against Helicobacter pylori. Antimicrob Agents Chemother 51, 551-559).

It has been also reported that a total mastic extract from *Pistacia lentiscus* exhibits antimicrobial activity against oral microorganisms (Karygianni, L., Cecere, M., Skaltsounis, A.L., Argyropoulou, A., Hellwig, E., Aligiannis, N., and Wittmer, A. (2014). High-level antimicrobial efficacy of representative Mediterranean natural plant extracts against oral microorganisms. Biomed Res Int, 839019). However, the null hypothesis of this report was that 24Z-isomasticadienolic acid has no antimicrobial effect on oral microbes.

Recent years have seen the increase in chemotherapeutic intransigence of microbial biofilms, whose antibiotic resistance is 1000 times higher compared to planktonic bacterial cells (Karygianni, L., Ruf, S., Follo, M., Hellwig, E., Bucher, M., Anderson, A.C., Vach, K., and Al-Ahmad, A. (2014). Novel Broad-Spectrum Antimicrobial Photoinactivation of In Situ Oral Biofilms by Visible Light plus Water-Filtered Infrared A. Appl Environ Microbiol 80, 7324-7336). In the oral cavity in particular, the antibiotic-resistant *E*. *faecalis* detected in infected root canals expressed the endocarditis-related antigen A (*EfaA*) (Al-Ahmad, A., Ameen, H., Pelz, K., Karygianni, L., Wittmer, A., Anderson, A.C., Spitzmuller, B., and Hellwig, E. (2014). Antibiotic resistance and capacity for biofilm formation of different bacteria isolated from endodontic infections associated with root-filled teeth. J Endod 40, 223-230). Due to the production of β-lactamases by *Prevotella* spp., *Fusobacterium* spp. and *Capnocytophaga* spp., an abundance of *^{bla}TEM* resistance genes could be identified in subgingival and tongue samples (Ioannidis, I., Sakellari, D., Spala, A., Arsenakis, M., and Konstantinidis, A. (2009). Prevalence of tetM, tetQ, nim and bla(TEM) genes in the oral cavities of Greek subjects: a pilot study. J Clin Periodontol 36, 569-574). Therefore, there is an urgent need for novel oral antimicrobials with low risk of provoking bacterial resistance to antibiotic monotherapy (Ngo, L.T., Okogun, J.I., and Folk, W.R. (2013). 21st century natural product research and drug development and traditional medicines. Nat Prod Rep 30, 584-592). In that context, the scenario of introducing novel phytopharmaceuticals has attracted attention lately (Jeon, J.G., Rosalen, P.L., Falsetta, M.L., and Koo, H. (2011). Natural products in caries research: current (limited) knowledge, challenges and future perspective. Caries Res 45, 243-263; Chandra Shekar, B.R., Nagarajappa, R., Suma, S., and Thakur, R. (2015). Herbal extracts in oral health care - A review of the current scenario and its future needs. Pharmacogn Rev 9, 87-92). The effectiveness of natural antimicrobial candidates can be attributed to their synergistic impact and broad pharmaceutical spectrum resulting from secondary metabolic reactions (Radulovic, N.S., Blagojevic, P.D., Stojanovic-Radic, Z.Z., and Stojanovic, N.M. (2013). Antimicrobial plant metabolites: structural diversity and mechanism of action. Curr Med Chem 20, 932-952; Yang, C., Chowdhury, M.A., Huo, Y., and Gong, J. (2015). Phytogenic compounds as alternatives to in-feed antibiotics: potentials and challenges in application. Pathogens 4, 137-156).

### Summary of the invention

The present invention provides 24Z-isomasticadienolic acid or a pharmaceutically acceptable salt thereof for use in the prevention or treatment of oral infections caused by oral microbes.

The present invention also provides oral care compositions comprising 24Z-isomasticadienolic acid or a pharmaceutically acceptable salt thereof.

### Detailed description of the invention

24Z-Isomasticadienolic acid is a natural product having the structure shown in formula (I)

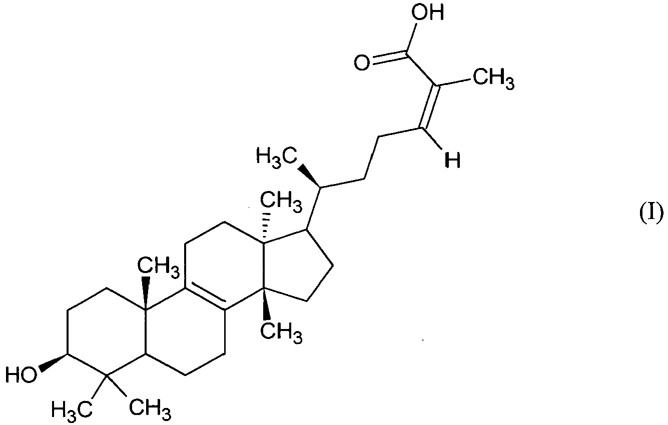

24Z-Isomasticadienolic acid can be isolated from *Pistacia lentiscus* L. (Anacardiaceae) (Paraschos, S., Magiatis, P., Mitakou, S., Petraki, K., Kalliaropoulos, A., Maragkoudakis, P., Mentis, A., Sgouras, D., and Skaltsounis, A.L. (2007). In vitro and in vivo activities of Chios mastic gum extracts and constituents against Helicobacter pylori. Antimicrob Agents Chemother 51, 551-559).

The antibacterial effects of 24Z-isomasticadienolic acid against *H. pylori* have already been reported. However, its activity against oral microbes has been surprising. According to the current state of the art, the oral cavity comprises 700-1200 different bacterial species that manage to survive under adverse conditions without oxygen and nutrients. *H. pylori* is not a representative member of this class of oral bacterial colonizers. All it does is to use the oral cavity as a springboard to penetrate the stomach where it can survive in its acidic environment. It has already been reported that *H. pylori* is transient in the oral cavity and although it may survive several hours in it, it fails to attach to the existing adherent microbial flora (Al-Ahmad, A., Kurschner, A., Weckesser, S., Wittmer, A., Rauberger, H., Jakob, T., Hellwig, E., Kist, M., and Waidner, B. (2012). Is Helicobacfer pylori resident or transient in the human oral cavity? J Med Microbiol 61, 1146-1152). There are two reasons for this: firstly, the fact that *H. pylori* is a bacterium with completely different growth demands than the representative oral bacteria and secondly, its great sensitivity in the hostile environment of the oral cavity makes its survival impossible after some days. *H. pylori* is much more susceptible to any treatment compared with the much more resistant oral bacteria. Therefore, if a drug can kill *H. pylori,* this does not mean that it shows an effective activity against oral bacteria.

Thus, the present invention provides 24Z-isomasticadienolic acid or a pharmaceutically acceptable salt thereof for use in the prevention or treatment of an oral infection caused by an oral microbe.

An oral microbe according to the present invention includes bacterial (Gram-positive, Gram-negative), viral, and fungal species. Preferably, the oral microbe is a Gram-positive bacterium, a Gram-negative bacterium or a yeast. More preferably, the oral microbe is *Streptococcus mutans, Streptococcus sobrinus, Streptococcus oralis*, *E. faecalis*, *S. aureus*, *Porphyromonas gingivalis*, *Fusobacterium nucleatum*, *Parvimonas micra* or *Candida albicans.*

The pharmaceutically acceptable salts of 24Z-isomasticadienolic acid include salts formed with inorganic or organic bases. Examples of such salts are ammonium salts, alkali metal salts such as sodium, lithium, and potassium salts, alkaline earth metal salts such as calcium and magnesium salts, salts with organic bases (for example, organic amines) or salts with amino acids such as arginine or lysine.

The present invention also provides an oral care composition comprising 24Z-isomasticadienolic acid or a pharmaceutically acceptable salt thereof.

The oral care composition of the present invention may be in various forms, such as toothpaste, dentifrice, gel, mouth rinse, mouth wash, solutions, mousse, foam, or mouth spray.

The oral care composition of the present invention typically comprises 24Z-isomasticadienolic acid or a pharmaceutically acceptable salt thereof and one or more additional ingredients well known to a person skilled in the art. Examples of additional ingredients include, among others, water, surfactants, polymers, thickening agents, buffering agents, fluoride ion sources, chelating agents, humectants, abrasives, agents for the increase of foaming and flavoring agents.

Examples of surfactants include, anionic, cationic, nonionic or zwitterionic surfactants. Examples of anionic surfactants include, among others, sodium lauryl sulfate, sodium lauroyl sarcosinate and sodium coconut monoglyceride sulfonates and mixtures thereof. Examples of cationic surfactants include, among others, lauryl trimethylammonium chloride, cetyl pyridinium chloride, cetyl trimethylammonium bromide, di-isobutylphenoxyethyldimethylbenzylammonium chloride, cetyl pyridinium fluoride and mixtures thereof. Examples of nonionic surfactants include, among others, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures thereof. Examples of zwitterionic surfactants include, among others, sodium alkyl sulfate, sodium lauroyl sarcosinate, cocoamidopropyl betaine and polysorbate 20 and mixtures thereof.

Examples of polymers include, among others, polyethylene glycols, polyvinylmethyl ether maleic acid copolymers, polysaccharides, polyacrylate gels, copolymers of maleic anhydride or acid with another polymerizable ethylenically unsaturated monomer, preferably methyl vinyl ether/maleic anhydride, copolymers of maleic anhydride with ethyl acrylate, hydroxyethyl methacrylate, N-vinyl-2-pyrollidone, or ethylene and mixtures thereof.

Examples of thickening agents include, among others, carboxyvinyl polymers, carrageenan, hydroxyethyl cellulose and water soluble salts of cellulose ethers such as sodium carboxymethyl cellulose and sodium carboxymethyl hydroxyethyl cellulose and mixtures thereof.

Examples of buffering agents include, among others, monosodium phosphate, trisodium phosphate, sodium benzoate, pyrophosphate salts, sodium lactate, sodium citrate and mixtures thereof.

Examples of fluoride ion sources include, among others, stannous fluoride, sodium fluoride, potassium fluoride, sodium monofluorophosphate, sodium fluorosilicate, ammonium fluorosilicate, amine fluoride, ammonium fluoride and mixtures thereof.

Examples of chelating agents include, among others, pyrophosphate salts, such as tetra alkali metal pyrophosphate, dialkali metal diacid pyrophosphate, trialkali metal monoacid pyrophosphate and mixtures thereof.

Examples of humectants include, among others, edible polyhydric alcohols such as glycerine, sorbitol, xylitol, propylene glycol or other polyols and mixtures thereof.

Examples of abrasives include, among others, a calcium phosphate abrasive, such as tricalcium phosphate, hydroxyapatite, dicalcium phosphate dihydrate or calcium pyrophosphate and mixtures thereof.

Examples of agents for the increase of foaming include, among others, polyethylene glycol and certain polymers, such as alginate polymers and mixtures thereof.

Examples of flavoring agents include, among others, essential oils, such as oils of spearmint, peppermint, wintergreen, sassafras, clove, sage, eucalyptus, marjoram, cinnamon, lemon, lime, grapefruit, orange, chemicals, such as menthol, carvone, or anethole and mixtures thereof.

The amount of 24Z-isomasticadienolic acid or of a pharmaceutically available salt thereof in an oral composition of the present invention is typically between 0.25% and 95% by weight. Preferably, the amount of 24Z-isomasticadienolic acid or of a pharmaceutically available salt thereof is between 0.25% and 65% by weight. More preferably, the amount of 24Z-isomasticadienolic acid or of a pharmaceutically available salt thereof is between 0.25% and 15% by weight. The term "% by weight" denotes the mass in grams of 24Z-isomasticadienolic acid or of a pharmaceutically available salt thereof per 100 grams of the composition.

The oral care compositions of the present invention can be prepared following processes well known to a person skilled in the art.

The oral care compositions of the present invention may be used for the prevention or treatment of oral infections caused by oral microbes. An oral infection caused by an oral microbe etiologically relates to pathological shifts in the supragingival and subgingival biofilms and can lead to the most common oral diseases such as caries, gingivitis, periodontal disease and periimplantitis (Karygianni, L., Ruf, S., Follo, M., Hellwig, E., Bucher, M., Anderson, A.C., Vach, K., and Al-Ahmad, A. (2014). Novel Broad-Spectrum Antimicrobial Photoinactivation of In Situ Oral Biofilms by Visible Light plus Water-Filtered Infrared A. Appl Environ Microbiol 80, 7324-7336). According to the Global Burden of Disease 2010 study, untreated dental caries in permanent teeth constituted the most prevalent disease across the globe, affecting 2.4 billion people, while untreated dental caries in deciduous teeth was found the tenth-most prevalent condition, affecting 621 million children worldwide (Kassebaum, N.J., Bernabé, E., Dahiya, M., Bhandari, B., Murray, C.J., Marcenes, W. (2015).Global burden of untreated caries: a systematic review and metaregression. J Dent Res 94, 650-658). Surprisingly, recent years have seen the increasing occurrence of periimplant inflammation ranging from 28 % to 56 %, especially in patients with history of periodontal disease (Schienle, S., Al-Ahmad, A., Kohal, R.J., Bernsmann, F., Adolfsson, E., Montanaro, L., Palmero, P., Fürderer, T., Chevalier, J., Hellwig, E., Karygianni, L. (2016). Microbial adhesion on novel yttria-stabilized tetragonal zirconia (Y-TZP) implant surfaces with nitrogen-doped hydrogenated amorphous carbon (a-C:H:N) coatings. Clin Oral Investig 20, 1719-1732).

### EXAMPLES

### Example 1

### Extraction from of 24Z-Isomasticadienolic acid from Pistacia lentiscus

24Z-Isomasticadienolic acid was isolated from mastic gum (Paraschos, S., Magiatis, P., Mitakou, S., Petraki, K., Kalliaropoulos, A., Maragkoudakis, P., Mentis, A., Sgouras, D., and Skaltsounis, A.L. (2007). In vitro and in vivo activities of Chios mastic gum extracts and constituents against Helicobacter pylori. Antimicrob Agents Chemother 51, 551-559). Conventional extraction of mastic gum for the preparation of total mastic extract without polymer (TMEWP) (extraction A) has already been described (Karygianni, L., Cecere, M., Skaltsounis, A.L., Argyropoulou, A., Hellwig, E., Aligiannis, N., and Wittmer, A. (2014a). High-level antimicrobial efficacy of representative Mediterranean natural plant extracts against oral microorganisms. Biomed Res Int, 839019). TMEWP partitioned between aqueous 5% Na₂CO₃ and ether as already described (extraction B) (Barton, D.H.R., and Seoane, E. (1956). Triterpenoids. Part XXII. The constitution and stereochemistry of masticadienonic acid. J Chem Soc 189, 4150-4157). The organic phase was re-extracted three times with 5 % Na₂CO₃ (extraction C) and afforded the neutral fraction of mastic (135 g) as the organic phase. The aqueous phase was added to that of extraction B and acidified with 1 N HCl. The acidic solution was reextracted with ether (extraction D), and the organic phase afforded the acid fraction of mastic (190 g).

The acidic fraction (20 g) was submitted to MPLC over normal-phase silica gel first with a cHex/CH₂Cl₂ gradient and then with a CH₂Cl₂/MeOH gradient affording 23 fractions. 24Z-isomasticadienolic acid was isolated by column chromatography over silica gel eluted with a CH₂Cl₂/MeOH gradient.

Identity and purity (≥95 %) of the isolated 24Z-isomasticadienolic acid were confirmed by NMR, MS and HPLC experiments and by comparison with literature data. All solvents (ethanol, methanol, dichloromethane and cyclohexane) were of p.A. quality. Water was purified by double distillation.

### Example 2

This example illustrates the activity of 24Z-isomasticadienolic acid against a number of oral microbes. More specifically, 24Z-isomasticadienolic acid was screened against eight representative bacterial inhabitants of the oral cavity, namely *Streptococcus mutans, Streptococcus sobrinus, Streptococcus oralis*, *Enterococcus faecalis, Porphyromonas gingivalis, Parvimonas micra, Fusobacterium nucleatum* and the yeast *Candida albicans.* Typical representatives of the skin and intestinal flora such as *Escherichia coli* and *Staphylococcus aureus* were used as reference bacteria. To assess the activity, two antimicrobial assays - the minimum bactericidal concentration (MBC) and the minimum inhibitory concentration (MIC) assay were applied.

### Bacterial strains and Candida albicans

A total of ten microbial strains from nine different bacterial strains and one *Candida albicans* strain were tested. Among these seven bacterial strains and C. *albicans* represent typical oral inhabitants, while the reference bacterial strains *Escherichia coli* and *Staphylococcus aureus* are members of the intestinal and skin flora, respectively. Their use facilitated the comparison of the antimicrobial activity of 24Z-isomasticadienolic acid within the oral cavity against its general inhibitory impact. Facultative anaerobic Gram-positive species such as *Streptococcus mutans* DSM 20523, *Streptococcus sobrinus* DSM 20381, *Streptococcus oralis* ATCC 35037, *Enterococcus faecalis* ATCC 29212 and S. *aureus* ATCC 25923 were tested. *Escherichia coli* ATCC 25922 served also as a facultative anaerobic bacterium but with a Gram-negative cell wall. The tested obligate anaerobes included *Porphyromonas gingivalis* W381, *Fusobacterium nucleatum* ATCC 25586 and *Parvimonas micra* ATCC 23195. The microorganisms were kept at -80 °C in basic growth medium containing 15 % (v/v) glycerol prior to use.

### Determination of the minimum inhibitory concentration (MIC)

As instructed in the Clinical and Laboratory Standards Institute (CLSI) guidelines an overnight culture of each bacterial strain and C. *albicans* was prepared and each dilution was placed on Columbia blood agar plates (CBA) or yeast-cysteine blood agar plates (HCB) (1999;2003). CBA agar plates were used for the incubation of facultative anaerobic bacteria and C. *albicans* at 37 °C and 5 % - 10 % CO₂ atmosphere for 24 h. HCB agar plates were used for the incubation of anaerobic bacteria at 37 °C for 48 h (anaerobic chamber, Genbox BioMérieux SA, Marcy/Etoile, France). For the microdilution assay at 10⁶ colony forming units (CFU) mL⁻¹ for each strain, Mueller-Hinton Broth (MHB) was utilized for the inoculation of all facultative anaerobic strains, Wilkins-Chalgren broth (WCB) for anaerobic bacteria and Sabouraud Dextrose Broth (SDB) for C. *albicans.* Then, with the aid of a multi-channel pipette appropriate volumes of the MHB/WCB/SDB microbial cultures were transferred into a 96-well microtiter-plate. Each well of the 96-well microtiter-plate had a total volume of 200 µL. Afterwards, 24Z-isomasticadienolic acid was dissolved in dimethyl sulfoxide (DMSO,) and diluted in distilled water. A concentration series ranging from 2500 µg mL⁻¹ to 2.4 µg mL⁻¹ at dilution levels starting from 2-fold to 512-fold was used to screen all 24Z-isomasticadienolic acid solutions in DMSO. The experiments were conducted in duplicate. For bacteria and fungi, a 0.5/1A McFarland standard suspension was diluted in normal saline. A dilution series of DMSO was tested in parallel in order to exclude potential inhibitory effects of the DMSO residuals. In case of bacterial growth in the co-tested DMSO dilution series the inhibitory impact of DMSO was taken into account. Wells containing only sterile MHB/WCB/SDB to minimize the possibility of contamination or 0.2 % chlorhexidine (CHX) served as positive and negative controls, respectively. Thereafter, facultative anaerobic bacteria and C. *albicans* were incubated at 37 °C and 5 % -10 % CO₂ atmosphere for 24 h, while anaerobic bacteria were kept at 37 °C for 48 h (anaerobic chamber, Genbox BioMérieux SA, Marcy / Etoile, France). All assays for each bacterial strain and *C*. *albicans* were conducted in duplicate. If the MIC values of a specific strain were not identical, the highest minimum inhibitory concentration (MIC) values were taken into account. MIC was determined as the lowest concentration of each compound at which visible inhibition of bacterial growth occurred, and was expressed by the percentage of bacterial growth at that particular concentration.

### Determination of the minimum bactericidal concentration (MBC)

As instructed in the CLSI guidelines the minimum bactericidal concentration (MBC) could also be determined ((1999). NCCLS, National Committee for Clinical Laboratory Standards Wayne, PA, USA; (2003). NCCLS, National Committee for Clinical Laboratory Standards Villanova, PA, USA). After the MIC testing, 10 µL from each well of the 96-well microtiter-plates containing the 24Z-isomasticadienolic acid concentration series were incubated on agar plates. In particular, Columbia blood agar (CBA) agar plates were used for facultative anaerobic bacteria and *C*. *albicans* at 37 °C and 5 % - 10 % CO₂ atmosphere for 2 days. Strictly anaerobic bacteria were incubated on yeast-cysteine blood agar (HCB) plates at 37 °C for 5 days (anaerobic chamber, Genbox BioMérieux SA, Marcy/Etoile, France). The colony forming units (CFU) were determined visually. The MBC was determined as the concentration at which a three log decrease in bacterial growth (= 99.9 %) was detected compared to the positive control.

### Results

Table 1 summarizes the MIC and MBC values of 24Z-isomasticadienolic acid for all screened microbial strains.

**Table 1. Antimicrobial activity in µg mL⁻¹ of 24Z-isomasticadienolic acid**

| | ***P. lentiscus*** | | | |
|---|---|---|---|---|
| | **24Z-isomasticadienolic acid** | | **DMSO** | |
| **Sample (% dilution in DMSO)** | **(3.9%)** | | **(in %)** | |
| **c/µg mL⁻¹** | **MIC*** | **MBC**** | **MIC** | **MBC** |
| *Streptococcus mutans* DSM 20523 | 78 | 156 | 6.00 | 25.00 |
| *Streptococcus sobrinus* DSM 20381 | 39 | 1250 | 10.00 | 15.00 |
| *Streptococcus oralis* ATCC 35037 | 39 | 78 | 10.00 | 10.00 |
| *Enterococcus faecalis* ATCC 29212 | 156 | 1250 | 15.00 | 25.00 |
| *Candida albicans* DSM 1386 | 1250 | 1250 | 8.00 | 8.00 |
| *Escherichia coli* ATCC 25922 | 1250 | 1250 | 10.00 | 10.00 |
| *Staphylococcus aureus* ATCC 25923 | 1250 | 1250 | 10.00 | 25.00 |
| *Porphyromonas gingivalis* W381 | 2.4 | 9.8 | 12.50 | 12.50 |
| *Fusobacterium nucleatum* ATCC 25586 | 625 | 625 | 6.25 | 6.25 |
| *Parvimonas micra* ATCC 23195 | 2.4 | 9.8 | 6.25 (5d) | 12.50 |

5d= values yielded after 5 days of incubation, *MIC = extract concentration at which the optical density (OD) measurement revealed minimal bacterial growth, **MBC = extract concentration at which a three log reduction (99.9 %) of the bacterial growth was induced.

24Z-Isomasticadienolic acid presented a substantial antimicrobial effect against the screened microorganisms. In its presence, a mean inhibitory concentration range of 2.4 µg mL⁻¹ (*P. gingivalis*, *P. micra*) to 625 µg mL⁻¹ (*F. nucleatum*) was observed for strict anaerobia. The MIC value for 24Z-isomasticadienolic acid was estimated between 39 µg mL⁻¹ (*S. sobrinus*, *S. oralis*) and 78 µg mL⁻¹ (*S. mutans*) for streptococci, while *E. faecalis* had a MIC value of 156 µg mL⁻¹. The highest MIC and MBC value of 24Z-isomasticadienolic acid (1.25 mg mL⁻¹) was detected for *E. coli*, *S*. *aureus* and *C*. *albicans.* The lowest MBC value (9.8 µg mL⁻¹) were determined for the obligate anaerobia *P*. *gingivalis* and *P*. *micra*, while 78 µg mL⁻¹ and 156 µg mL⁻¹ of the compound killed 99.9% of *S*. *oralis* and *P*. *micra* and *S*. *mutans*, respectively.

The results of table 1 demonstrate that 24Z-isomasticadienolic acid exhibits a substantial and unexpected antimicrobial activity against both Gram-positive and Gram-negative anaerobic oral pathogens as well as streptococci.

## Claims

1. 24Z-Isomasticadienolic acid or a pharmaceutically acceptable salt thereof for use in the prevention or treatment of an oral infection caused by an oral microbe.

2. 24Z-Isomasticadienolic acid or a pharmaceutically acceptable salt thereof according to claim 1 wherein the oral microbe is selected from the group consisting of a Gram-positive bacterium, a Gram-negative bacterium and a yeast.

3. 24Z-Isomasticadienolic acid or a pharmaceutically acceptable salt thereof according to claim 1 or 2, wherein the oral microbe is selected from the group consisting of *Streptococcus mutans, Streptococcus sobrinus, Streptococcus oralis*, *Enterococcus faecalis, S*. *aureus, Porphyromonas gingivalis*, *Fusobacterium nucleatum, Parvimonas micra* and *Candida albicans.*

4. 24Z-Isomasticadienolic acid or a pharmaceutically acceptable salt thereof for use in the prevention or treatment of an oral disease selected from the group consisting of caries, gingivitis, periodontal disease and periimplantitis.

5. Oral care composition comprising 24Z-isomasticadienolic acid or a pharmaceutically acceptable salt thereof.

6. Oral care composition according to claim 5, for use in the prevention or treatment of an oral infection caused by an oral microbe.

7. Oral care composition according to claim 6, wherein the oral microbe is selected from the group consisting of a Gram-positive bacterium, a Gram-negative bacterium and a yeast.

8. Oral care composition according to claim 7, wherein the oral microbe is selected from the group consisting of *Streptococcus mutans, Streptococcus sobrinus, Streptococcus oralis, Enterococcus faecalis, Staphylococcus aureus, Porphyromonas gingivalis, Fusobacterium nucleatum, Parvimonas micra* and *Candida albicans.*

9. Oral care composition according to any one of claims 4, for use in the prevention or treatment of an oral disease selected from the group consisting of caries, gingivitis, periodontal disease and periimplantitis.

10. Oral care composition according to any one of claims 4 to 9, which is in a form selected from the group consisting of toothpaste, dentifrice, gel, mouth rinse, mouth wash, solution, mousse, foam, and a mouth spray.

11. Oral care composition according to any one of claims 4 to 10, comprising from 0.25% to 95% by weight of 24Z-isomasticadienolic acid or a pharmaceutically acceptable salt thereof.

12. Oral care composition according to any one of claims 4 to 10, comprising from 0.25% to 65% by weight of 24Z-isomasticadienolic acid or a pharmaceutically acceptable salt thereof.

13. Oral care composition according to any one of claims 4 to 10, comprising from 0.25% to 15% by weight of 24Z-isomasticadienolic acid or a pharmaceutically acceptable salt thereof.
